# EUROPÄISCHE PATENTSCHRIFT

(11) **EP 1 235 564 B1**
(45) Veröffentlichungstag und Bekanntmachung des Hinweises auf die Patenterteilung: **03.05.2006**
(21) Anmeldenummer: 00977571.9
(22) Anmeldetag: 25.11.2000
(51) Int. Cl.: A61K 31/02, A61K 33/18

(54) **TRIIODMETHAN-VERDICKERKONJUGATE, VERFAHREN ZU IHRER HERSTELLUNG SOWIE VERWENDUNG ALS DESINFEKTIONSREINIGER ZUR REINIGUNG UND PFLEGE**
TRIIODOMETHANE THICKENER CONJUGATES, A METHOD FOR THEIR PRODUCTION AND THEIR USE AS DISINFECTANT CLEANERS AND HYGIENE PRODUCTS
CONJUGUES D'EPAISSISSEMENT A BASE DE TRIIODOMETHANE, LEUR PROCEDE DE PRODUCTION ET LEUR UTILISATION EN TANT QUE PRODUIT DE NETTOYAGE DESINFECTANT UTILISE POUR NETTOYER ET ENTRETENIR

(30) Priorität: 25.11.1999 DE 19957918
(43) Veröffentlichungstag der Anmeldung: 04.09.2002
(73) Patentinhaber: Doht, Ulrich, 21224 Rosengarten (DE)
(72) Erfinder: Doht, Ulrich, 21224 Rosengarten (DE)
(74) Vertreter: Wenzel & Kalkoff
(86) Internationale Anmeldenummer: PCT/EP2000/011738
(87) Internationale Veröffentlichungsnummer: WO 2001/037822

(56) Entgegenhaltungen:
- EP-A- 0 244 118
- EP-A- 0 258 761
- EP-A- 0 487 066
- WO-A-96/40086
- GB-A- 2 048 070
- DATABASE WPI Week 199945 Derwent Publications Ltd., London, GB; AN 1997-389348 XP002164239 & JP 02 953649 B (IWAKI SEIYAKU KK), 27. September 1999 (1999-09-27)

## Beschreibung

Die Erfindung betrifft ein Konjugat aus Triiodmethan und Wasserverdickern, ein dieses Konjugat enthaltendes Hydrogel, die Verwendung dieser Verbindungen sowie ein Verfahren zu ihrer Herstellung.

Bestimmte Iodverbindungen, die Iod in der Oxidationsstufe 0 oder -1 enthalten, sind dafür bekannt, mikrobizid zu wirken. Dementsprechend finden solche Verbindungen Anwendung in Desinfektionsmitteln. Elementares Iod, meist in Form eines Polyvinylpyrrolidon-Konjugats, hat sich beispielsweise sehr erfolgreich als Antiseptikum zur Wundversorgung etabliert. Die Hornpflege im Rahmen der Stalltierhaltung, insbesondere von Pferden, Rindern und Schafen, erfordert neben Desinfektionsmitteln noch weitere Maßnahmen. Das Horn der Hufsohlen (Hautanhangsgebilde) dieser Tiere bedarf einer Pflege mit feuchtigkeitsbindenden Mitteln, da unbehandeltes Horn die Neigung hat, Risse zu bilden, und signifikant an Elastizität verliert. Über diese Risse und Hornspalten können massiv hornzersetzende Fäkalbakterien in die Hufsohle eindringen.

Diese hornzersetzenden Darmbakterien *(Fusobakterii necrophori)* sammeln sich zusammen mit Jauche im Stalluntergrund (Stroh bzw. Holzspäne) an und vermehren sich dort unter anaeroben Bedingungen und greifen die Tierhufe an. Zur Pflege und Reinigung von Tierhufen ist es dabei bekannt, diese mit Salben auf Zinkoxid-Basis zu behandeln. Bei massivem Befall der Hufsohlen werden sie am besten äußerlich mit antibakteriell wirksamen Mitteln bekämpft. Allgemein bekannte Mittel hierfür sind z.B. 1-5%iges Wasserstoffperoxid, 5-10% Kupfer(II)-Verbindungen oder 1-5% igem Formaldehyd in Wasser bzw. Alkoholen. Das bisher wirksamste verfügbare Mittel ist 4-10%iges Triiodmethan in Diethylether (s. "Richtlinien für Reiten und Fahren", 2. Aufl., Bd. VI (Pferdehaltung), S. 128, FN-Verlag, Warendorf 1980). Üblicherweise sind zur effektiven Bekämpfung von Bakterien jedoch hydrophile Bedingungen in wirksamen Desinfektionsmitteln notwendig.

Anaerobe Bakterien sind auch dazu geeignet, neben Polypeptiden (Hufhom) auch andere organische Makromoleküle (z.B. Celluloseether) zu degenerieren. Wasserstoffperoxid, Formaldehyd, Kupfer(II)-Ammoniak-Komplexe und Zinkchlorid sind für Abbau-, Vernetzungs- und Komplexierungsreaktionen mit organischen Verdickern (z.B. Celluloseethern) bekannt.

Aus der Humanmedizin ist ein mittelviskoses, wassermischbares Iod-Adsorbat mit Polyvinylpyrrolidon (PVP) als Antiseptikum (z.B. Betaisodona®) bekannt. Polyvinylpyrrolidon wird als vollsynthetisches organisches Verdickungsmittel bezeichnet. Dieses Mittel weist jedoch gravierende Nachteile auf, die es für einen Einsatz als Hufpflegemittel ungeeignet machen. Dabei ist zum einen der hohe Preis zu nennen. Des weiteren hat Betaisodona® den Nachteil, sich bei Körpertemperatur zu verflüssigen. Ein Einsatz im Rahmen der Stalltierhaltung kommt somit nicht in Frage, da bei Anwendung des Produktes auf Tierhufen bzw. -klauen sofort die genannte Verflüssigung eintritt, so daß das Mittel nicht auf der aufgetragenen Stelle haftet. Des weiteren ist die Feuchtigkeitszufuhr im Rahmen der Hufpflege von zentraler Bedeutung. Betaisodona® enthält bei weitem nicht genug Wasser, um den Tierhuf ausreichend versorgen zu können. Eine einfache Untermischung von zusätzlichem Wasser ist jedoch nicht möglich, da das Produkt dann flüssig werden würde und eine einfache Anwendung nicht mehr gewährleisten könnte.

Abgewandelte natürlich-organische Verdicker werden häufig als Gelbildner für dünnflüssige alkoholhaltige (schnell abtrocknende) Gele benutzt, die durch einen geringen Mikrobizid-Gehalt desinfizierend wirkende Filme auf der Haut hinterlassen. In der EP 0 640 352 wird Ethylcellulose eingesetzt, andere Celluloseether sind hierfür ebenfalls geeignet (WO 99/43205). Neben Iod bzw. Iodophoren wie PVP oder Iod-Stärke-Komplexen können auch andere anitmikrobiell wirkende Substanzen verwendet werden.

Alle bisher zur Pflege oder Desinfektion von Hufsohlen bekannten Mittel weisen große Nachteile auf. So sind sie insbesondere zu niederkkos, nur schwierig handhabbar und wegen fehlender Haftwirkung nur ungenügend wirksam. Ein unter der Bezeichnung "Chevaline- Strahlpflegepaste" vertriebenes Produkt besteht aus Bolus Alba und Isopropanol und enthält als Wirkstoff eine organische Kupfer(II)verbindung. Dieses Produkt ist jedoch nicht homogen und hat eine eher bröckelige Konsistenz. Es ist daher nur schwierig zu applizieren. Ferner weist es den gravierenden Nachteil auf, daß Kupfersalze erfahrungsgemäß Horn verhärten. Das grundsätzlich sehr wirksame Triiodmethan in Diethylether hingegen ist aufgrund der hohen Flüchtigkeit nicht nur schwierig zu handhaben, sondern zudem aufgrund der Entflammbarkeit nicht ungefährlich. Auch ist die Dosierung durch die Flüchtigkeit sehr schwierig, und eine schnelle Anwendung kann nicht gewährleistet werden.

Insgesamt haben sich Iodverbindungen als besonders wirksame Mikrobizide in Desinfektionsmitteln herausgestellt. Die bislang bekannten Verbindungen weisen allerdings für das Anwendungsgebiet der Hornpflege von Tierhufen die angeführten gravierenden Nachteile auf.

Die Aufgabe der vorliegenden Erfindung besteht daher darin, insbesondere in Verbindung mit Reinigungs- und Pflegemitteln eine Iodverbindung zur Verfügung zu stellen, die eine antibakterielle Wirkung aufweist, einfach zu handhaben ist sowie schnell und exakt aufgebracht werden kann. Weiterhin sollte sie ein gutes Haftvermögen und eine ausreichende Wasserlöslichkeit aufweisen.

Erfindungsgemäß wird diese Aufgabe durch ein Hydrogel gelöst, das ein Konjugat aus Triiodmethan und mindestens einem natürlich-organischen und/oder modifiziert natürlich-organischen und/oder an organischen Verdickern sowie Wasser und einem Lösevermittler enthält, wobei das Hydrogel eine Viskosität zwischen 10.000 und 30.000 mPa*s(20°C) aufweist. Im Rahmen der Erfindung wirde überraschenderweise gefunden, daß Triiodmethan mit Verdickern, vorzugsweise mit anorganischen und organischen, insbesondere mit natürlich-organischen und modifizierten natürlich-organischen Verdickern, Konjugate bildet. Anders als bei allen bisher bekannten Verbindungen wirken die Verdicker dabei nicht nur als Gelbildner im Wasser, sondern auch als Komplexbildner für das Triiodmethan.

Als Verdicker werden vorzugsweise modifizierte natürlich-organische Makromoleküle wie Celluloseether oder Stärkeether eingesetzt. Vorzugsweise kommen Methylcellulose, Ethylcellulose, Methylhydroxyethylcellulose, Methylhydroxypropylcellulose, Hydroxyethylcellulose, Hydroxypropylcellulose, Ethylhydroxyethylcellulose, Carboxymethylcellulose, Hydroxypropylstärkeether, Hydroxyethylstärkeether oder Carboxymethylstärkeether zum Einsatz, wobei Methylcellulose, Methylhydroxypropylcellulose, Carboxymethylcellulose und Hydroxypropylstärkeether besonders geeignet sind.

Als Verdicker sind weiterhin natürlich-organische Makromoleküle wie handelsübliche Lebensmittelprodukte besonders geeignet, vorzugsweise Agar-Agar, Guarkernmehl, Johannisbrotbaum-Kernmehl und Stärke, wobei Johannisbrotbaum-Kernmehl besonders geeignet ist.

Ebenfalls gut geeignet sind anorganische Makromoleküle wie beispielsweise handelsübliche Polykieselsäuren oder Tonmineralien, vorzugsweise Bentonit.

Die in dem Hydrogel enthaltenen Konjugate können gefüllt werden. Diese gefällten Konjugate eignen sich hervorragend zur Einbringung in Salben, Cremes, Gelen und Pasten. Sowohl die erfindungsgemäßen Hydrogele wie auch die Konjugate in der zuvor genannten Form sind insbesondere für die Verwendung als wirksamer Desinfektionsreiniger zur Reinigung und Pflege für Warmblüter, insbesondere als Hompflegemittel für Pferdehufe und Klauen bei Rindern und Schafen, geeignet. In einer solchen Form werden die an die Erfindung gestellten Aufgaben erfüllt: Einfache Handhabung und genaue und schnelle Aufbringung. Das Mikrobizid wirkt dabei nicht nur auf dem zu behandelnden Horn antibakteriell, sondern schließt auch bakterielle Verunreinigungen, die anwendungsbedingt während der Hufpflege unvermeidbar in den Desinfektionsreiniger eingemischt werden, aus.

Insbesondere die Verbindungen von Celluloseethern und Trüodmethan stellen sich als sehr geeignete Mittel zur Hornpflege für Warmblüter heraus, zum einen in Form einer das oben erwähnte Konjugat enthaltender Salbe oder Creme, zum anderen in Form von Hydrogelen.

Bevorzugte Lösevermittler für das Hydrogel sind Glycolether, insbesondere Monomethyl-, Monoethyl-, Monobutyl-, Dimethyl-, Diethyl- oder Dibutylether von Ethylenglycol, Diethylenglycol, Triethylenglycol, Propylenglycol, Dipropylenglycol oder Tripropylenglycol, insbesondere Dipropylenglycol-mono-methylether oder Dipropylenglycoldimethylether. Der Anteil der wasserlöslichen Lösevermittler beträgt vorzugsweise 0,5 bis 30 Gew.-%, insbesondere 2 bis 10 Gew.-%, bezogen auf das Gel.

Der Anteil der modifizierten organischen Verdicker beträgt vorzugsweise 0,5 bis 20 Gew.-%, insbesondere 1 bis 15 Gew.-%, bezogen auf das Gel. Der Anteil der natürlichen organischen Verdicker beträgt vorzugsweise 0,3 bis 15 Gew.-%, insbesondere 0,5 bis 10 Gew.-%, bezogen auf das Gel. Der Anteil der anorganischen Verdicker beträgt vorzugsweise 5 bis 25 Gew.-%, insbesondere 10 bis 20 Gew.-%, bezogen auf das Gel.

Der Anteil des Triiodmethans beträgt vorzugsweise 0,01 bis 20 Gew.-%, insbesondere 0,1 bis 15 Gew.-%, bezogen auf das Gel.

Für die Hufpflege ist es von großer Bedeutung, den Huf vor dem Austrocknen zu schützen. Daher sollte das Gel für seine Anwendung als Desinfektionsreiniger zur Reinigung und Pflege von Horn ein hohes Rückhaltevermögen für Wasser aufweisen, um einem Austrocknen entgegenzuwirken.

In einer besonders vorteilhaften Ausführungsform enthält das Gel daher weiterhin mindestens einen Polyalkohol, vorzugsweise Ethylenglycol, Diethylenglycol, Triethylenglycol, 1,2-Propylenglycol, Dipropylenglycol, Tripropylenglycol und Glycerin, wobei Diethylenglycol, Dipropylenglycol und Glycerin besonders geeignet sind. Der Anteil der Polyalkohole beträgt vorzugsweise 0.01 bis 40 Gew.-%, insbesondere 5 bis 30 Gew.-%, bezogen auf das Gel.

Durch den Zusatz von Glycerin kann das Gel sehr geschmeidig eingestellt werden. Glycerin erhöht außerdem zusätzlich das Wasserrückhaltevermögen des Gels, so daß die Substanz als Pflegemittel besonders geeignet ist. Dies ist wichtig und bei bisher bekannten Desinfektionsmitteln, z.B. dem Trüodmethan in Diethylether, nicht gegeben. Letztere Verbindung verdampft sehr leicht, d.h. trocknet schnell ab, und bewirkt ein Austrocknen des Horns. Es sorgt jedoch nicht dafür, daß Wasser im Horn gehalten wird, um diesem Geschmeidigkeit zu verleihen. Das Gel trocknet hingegen nur sehr langsam ab und kann dadurch eine besondere Pflegewirkung entfalten.

Das Gel kann weiterhin Zusatzstoffe wie andere pflegende Komponenten aus der Gruppe der langkettigen Ester, vorzugsweise Triglyceride, insbesondere Lorbeeröl *(Lauri Oleum)* enthalten. Der Anteil der Zusatzstoffe beträgt vorzugsweise 1 bis 10 Gew.-%, insbesondere 3 bis 8 Gew.-%, bezogen auf das Gel. Auch können dem Gel Parfümöle zugesetzt werden. Der Anteil solcher Parfümöle beträgt vorzugsweise 0,01 bis 0,1 Gew.-%.

Die vorliegende Erfindung betrifft des weiteren auch ein Verfahren zur Herstellung von solchen Hydrogelen. Dabei wird zunächst auf übliche Weise ein hochviskoses Hydrogel hergestellt. Die einzuhaltende Wassertemperatur richtet sich nach der Art des verwendeten Verdickers. Dieser Lösung werden anschließend bei 20-60°C in folgender Reihenfolge unter intensivem Rühren zunächst der Polyalkohol und schließlich in einem Teil des Lösevermittlers das Mikrobizid, Triiodmethan, zugesetzt. Zweckmäßigerweise wird danach bei einer Temperatur von 20-40°C, vorzugsweise bei Raumtemperatur, homogenisiert.

Die Konjugate, die das Triiodmethan mit den Verdickern bildet, können bei hoher Kochsalzkonzentration und/oder hoher Wasserverdünnung gefällt werden. Geprüft wurden Verdünnungen der Gele mit der bis zu 12fachen Menge an Wasser bei 0%, 5% und 10% Kochsalzzusatz. Besonders geeignet als Verdicker sind dabei Celluloseether und Stärken.

Die Gele zeigen aufgrund ihrer Viskosität ausgezeichnete Hafteigenschaften und sehr gute Wirksamkeit als Desinfektionsreiniger auf Horn von Tierhufen, wie sie von bisher bekannten Mitteln nicht erreicht werden. Sie können für den angestrebten Anwendungsbereich ohne weitere Hilfsmittel mit einem Pinsel aufgenommen und einfach in Hornspalten von Tierhufen plaziert werden. Durch die Wasserrückhaltung der verwendeten Verdickungsmittel wird eine Austrocknung des Horns nachhaltig verhindert und bereits aufgeweichtes Horn wieder deutlich stabilisiert. Gleichzeitig wirken bei der Auftragung mit einem Pinsel oberflächenaktive Verdickungsmittel als nichtionische Tenside (Schaumbildung). Erstaunlicherweise kann so bereits nach drei- bis fünfmaliger Anwendung in den Strahlfurchen sogar eine Strahlfäule-Ausbreitung auf Pferdehufen gestoppt werden. Bakterielle Stall-Verunreinigungen, die infolge häufiger Benutzung in das Gel eingetragen werden, reduzieren die Viskosität des Gels nicht.

Das Gel kann auch in anderen, z.B. allgemein medizinischen Anwendungsbereichen eingesetzt werden. Dabei ist insbesondere ein Einsatz in der Hautbehandlung sowie in der Wundversorgung zu nennen.

Weitere Vorteile, Ausführungsformen und Anwendungsmöglichkeiten gehen aus den nachfolgend angeführten Beispielen hervor.

Es wurden Hydrogele unterschiedlicher Zusammensetzung hergestellt. Die eingesetzten Substanzen sind der nachfolgenden Tabelle 1 zu entnehmen. In einem Vergleichsversuch wurde anstelle eines anorganischen, eines natürlich-organischen oder eines abgewandelt natürlich-organischen Verdickers ein vollsynthetisch-organischer Verdicker gewählt. Als Polyalkohol wurde Glycerin verwendet. Als Lösevermittler wurden Dipropylenglycol-dimethyl- und -monomethylether eingesetzt. Die Herstellung der hochviskosen Hydrogelen erfolgt nach allgemein bekannten Methoden. Die einzuhaltenden Wassertemperaturen richten sich nach der Art des verwendeten Verdickers und sind ebenfalls in der nachstehenden Tabelle 1 aufgeführt.

Den Verdicker-Lösungen werden anschließend bei 20-60°C unter intensivem Rühren die weiteren Komponenten in folgender Reihenfolge zugesetzt:
a) Polyalkohol
b) Lösevermittler (Dipropylenglycoldimethylether und/oder - monomethylether)
c) Mikrobizid.

Das Mikrobizid wird in einem Teil des zu verwendenden Glycolethers gelöst zugesetzt. Es wird zweckmäßigerweise danach bei Raumtemperatur homogenisiert. Die erfindungsgemäßen Gele weisen Viskositäten von 5.000 bis 400.000 mPa*s auf.

Bei Verwendung von Triiodmethan mit organischen Verdickern bilden sich hierbei stets zitronengelb gefärbte wäßrige Triiodmethan-Adsorbate (Konjugate). Die Lösungen trocknen auf einer Glasplatte zu transparenten Filmen mit sehr hoher Elastizität aus.

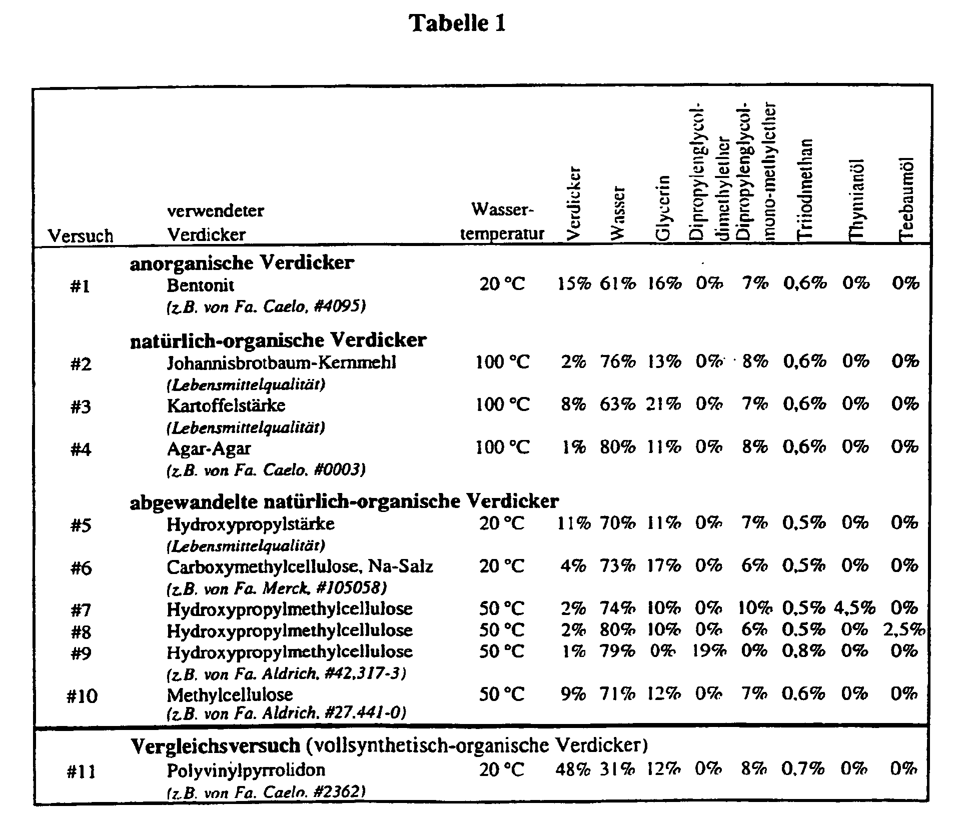

Aus dem Vergleichsversuch mit Polyvinylpyrrolidon ist erkennbar, daß dies für das erfindungsgemäße Einsatzgebiet weniger gut geeignet ist. Um eine den anderen Produkten entsprechende Viskosität zu erreichen, ist ein erheblich höherer Anteil Verdicker erforderlich. Dafür kann nur maximal die Hälfte des Wassergehaltes erreicht werden. Da ein wichtiger Aspekt des erfindungsgemäßen Desinfektionsreinigers dessen Wassergehalt und die damit verbundene pflegende Wirkung ist, wird deutlich, daß vollsynthetisch-organische Verdicker zum erfindungsgemäßen Einsatz wenig geeignet sind.

Getestet wurde eine zweimonatige Dauereinwirkung des Gels auf das Hufhorn. Die Anwendung des Gels beeinflußt die Eigenschaften von Hufhom nicht negativ. Hierzu wurde ein frisch geschnittenes Homstück geteilt und in Wasser sowie in ein erfindungsgemäßes Trüodmethan-haltiges Gel eingelegt. Nach zwei Monaten wurden beurteilt: Elastizität des Horns, Zersetzungsprodukte, Verfärbung. Das Ergebnis ist in der nachfolgenden Tabelle 2 dargestellt:

**Tabelle 2**

| | | |
|---|---|---|
| | in Gel | in Wasser |
| | | |
| Elastizität des Horns | unverändert | bröckelig |
| Verfärbung des Horn | keine | dunkler |
| Zersetzungsprodukte | keine | schwarze Schwebstoffe; fauliger Geruch des Wassers |

Die Herstellung der Trüodmethan-Verdicker-Konjugate erfolgt durch Ausfällen der gemäß dem obigen Ausführungsbeispiel hergestellten Hydrogele mittels hoher Kochsalzkonzentration und/oder hoher Wasserverdünnung. Dabei werden die erfindungsgemäßen Gele mit der bis zu 12fachen Menge an Wasser bei 0%, 5% und 10% Kochsalzzusatz verdünnt.

Aus der Färbung der Lösung und aus der Menge des gleichmäßig gefärbten Niederschlags kann das Adsorptionsvermögen des verwendeten Verdickers gegenüber Triiodmethan beurteilt werden. Zum Vergleich dient die Fällung aus einer Verdicker-freien Lösung; hierbei entsteht ein feindisperser zitronengelber Niederschlag von Triiodmethan und eine farblose wäßrige Phase. Das Ergebnis ist in der folgenden Tabelle 3 dargestellt:

**Tabelle 3**

| Adsorptionsvermögen des Makromoleküls | Löslichkeit des Adsorbats (Konjugats) | Makromolekül (Verdicker) |
|---|---|---|
| schlecht | ----- | Polyvinylpyrrolidon |
| | gut | Kartoffelstärke |
| mittel | gut | Stärken (Hydroxypropyl-subst.) |
| | gut | Methylcellulosen (Hydroxypropyl- oder Carboxy-subst.) |
| mittel | mittel | Methylcellulose |
| gut | schlecht | Agar-Agar, Johannisbrotbaum-Kemmehl Bentonit |

Bei hoher Verdünnung der Gele fallen in Wasser bei bis zu 10% Kochsalzgehalt die Konjugate von Triiodmethan und verschiedenen Verdickern als mehr oder weniger voluminöse Niederschläge aus. Schlecht lösliche Konjugate können bereits durch eine geringe Wasserverdünnung gefällt werden, gut lösliche Konjugate dagegen erst in hoher Wasserverdünnung unter gleichzeitiger Verwendung von Kochsalz.

Im Gegensatz zu reinem Trüodmethan sind die Triiodmethan-Konjugate in Dialkylethern wie beispielsweise Diethylether oder Methyl-tert.-butylether schwerlöslich. Die Konjugate trocknen auf einer Glasplatte zu einem milchigtrüben, spröden Film aus.

Die isolierten Niederschläge enthalten die gesamte Trüodmethan-Menge in feindispers verteilter Form neben wechselnden Mengen an Verdicker und Wasser. Diese Niederschläge können unter Berücksichtigung des Wassergehalts einfach in handelsüblichen pharmazeutischen Basismaterialien, beispielsweise wasserhaltiger hydrophiler Salbe *(Unguentum emulsificans aquosum;* DAB 1999), hydrophiler Salbe *(Unguentum emulsificans;* DAB 1999), Glycerinsalbe *(Unguentum glycerini:* DAB 6) oder Basiscreme *(Cremor basalis,* DAC 1997) verteilt werden. Hierzu werden voluminöse, stark wasserhaltige Niederschläge (beispielsweise Konjugate mit Agar-Agar, Methylcellulose, Bentonit) in hydrophiler Salbe oder Glycerinsalbe verteilt. Kompakte Niederschläge (beispielsweise Konjugate mit Hydroxypropylmethylcellulose, Johannisbrotbaum-Kernmehl, Stärke) können dagegen auch in Basiscreme oder wasserhaltiger hydrophiler Salbe dispergiert werden. Die Viskosität kann mit Wasser und/oder Glycerin nachgestellt werden. Die folgende Tabelle 4 gibt eine Übersicht über mögliche Produkte.

**Tabelle 4**

| Triiodmethan-Konjugat mit | | verwendete Salben-bzw. Creme-Grundlage |
|---|---|---|
| Stärke | kompakt, geringer Wassergehalt | wasserhaltige hydrophile Salbe (*z.B. von Fa. Caelo #3106)* |
| Methylcellulose | voluminös, hoher Wassergehalt | hydrophile Salbe *(z.B. von Fa. Caelo #3104)* |
| Methylcellulose | voluminös, hoher Wassergehalt | Glycerinsalbe (*z.B. von Fa. Caelo #3114*) |
| Agar-Agar | voluminös, sehr hoher Wassergehalt | Basiscreme *(z.B. von Fa. Caelo #3013*) |

Triiodmethan-Konjugate mit Verdickern können daher ganz allgemein als Konservierungsmittel in Salben, Cremes, Gelen und Pasten dienen. In den Beispielen beträgt der Triiodmethangehalt 0.5% und es können andere hornpflegende Zusatzstoffe wie beispielsweise festes Lorbeeröl *(Oleum Lauri expressum,* DAB 6) eingemischt werden. Diese Gemische eignen sich wie die oben genannten Gele ebenfalls als Hompflegemittel.

## Patentansprüche

1. Hydrogel, enthaltend ein Konjugat aus Triiodmethan und mindestens einem natürlich-organischen und/oder modifiziert natürlich-organischen und/oder anorganischen Verdicker sowie Wasser und einen Lösevermittler, wobei das Hydrogel eine Viskosität zwischen 5.000 und 400.000 mPa*s (20°C), vorzugsweise zwischen 10.000 und 30.000 mPa*s (20°C), aufweist.

2. Hydrogel nach Anspruch 1, **dadurch gekennzeichnet, daß** der Verdicker ein modifizierter natürlich-organischer Verdicker, insbesondere ein Celluloseether oder Stärkeether, vorzugsweise Methylcellulose, Ethylcellulose, Methylhydroxyethylcellulose, Methylhydroxypropylcellulose, Hydroxyethylcellulose, Hydroxypropylcellulose, Ethylhydroxyethylcellulose, Carboxymethylcellulose, Hydroxypropylstärkeether, Hydroxyethylstärkeether oder Carboxymethylstärkeether, ist.

3. Hydrogel nach Anspruch 1, **dadurch gekennzeichnet, daß** der Verdicker ein natürlich-organischer Verdicker, insbesondere Agar-Agar, Guarkernmehl, Johannisbrotbaum-Kemmehl oder Stärke, ist.

4. Hydrogel nach Anspruch 1, **dadurch gekennzeichnet, daß** der Verdicker ein anorganischer Verdicker, insbesondere eine Polykieselsäure oder ein Tonmineral, vorzugsweise Bentonit, ist.

5. Hydrogel nach einem der Ansprüche 1 bis 4, **dadurch gekennzeichnet, daß** der Ltisevermittler ein Glycolether, insbesondere ein Monomethyl-, Monoethyl-, Monobutyl-, Dimethyl-, Diethyl- oder Dibutylether von Ethylenglycol, Diethylenglycol, Triethylenglycol, Propylenglycol, Dipropylenglycol oder Tripropylenglycol, insbesondere Dipropylenglycol-mono-methylether oder Dipropylenglycoldimethylether, ist.

6. Hydrogel nach einem der Ansprüche 1 bis 5, **dadurch gekennzeichnet, daß** das Hydrogel im Falle eines natürlich-organischen Verdickers 0,3 bis 15 Gew.%, vorzugsweise 0,5 bis 10 Gew.%, Verdicker, im Falle eines modifiziert natürlich-organischen Verdickers 0,5 bis 20 Gew.-%, vorzugsweise 1 bis 15 Gew.%, Verdicker und im Falle eines anorganischen Verdickers 5 bis 25 Gew.-%, vorzugsweise 10 bis 20 Gew.%, Verdicker, 0,5 bis 30 Gew.%, vorzugsweise 2 bis 10 Gew.%, Lösevermittler und 0,01 bis 20 Gew.-%, vorzugsweise 0,1 bis 15 Gew.-%, Trüodmethan enthält.

7. Hydrogel nach einem der Ansprüche 1 bis 6, **dadurch gekennzeichnet, daß** das Hydrogel des weiteren mindestens einen Polyalkohol, insbesondere Ethylenglycol, Diethylenglycol, Triethylenglycol, 1,2-Propylenglycol, Dipropylenglycol, Tripropylenglycol oder Glycerin, enthält.

8. Hydrogel nach Anspruch 7, **dadurch gekennzeichnet, daß** das Hydrogel 0,01 bis 40 Gew.-%, vorzugsweise 5 bis 30 Gew.-%, Polyalkohol enthält.

9. Hydrogel nach einem der Ansprüche 1 bis 8, **dadurch gekennzeichnet, daß** das Hydrogel des weiteren 1 bis 10 Gew.-%, vorzugsweise 3 bis 8 Gew.-%, eines langkettigen Esters, insbesondere eines Triglycerids, vorzugsweise Lorbeeröl, enthält.

10. Hydrogel nach einem der Ansprüche 1 bis 9, **dadurch gekennzeichnet, daß** das Hydrogel des weiteren 0,01 bis 0,1 Gew.-% Parfümöle enthält.

11. Konjugat aus Trüodmethan und mindestens einem natürlich-organischen und/oder modifiziert natürlich-organischen und/oder anorganischen Verdicker, erhältlich durch Fällung des Hydrogels nach einem der Ansprüche 1 bis 10.

12. Verwendung des Hydrogels nach einem der Ansprüche 1 bis 10 oder des Konjugats nach Anspruch 11 zur Herstellung eines Desinfektionsmittels.

13. Verwendung des Hydrogels nach einem der Ansprüche 1 bis 10 oder des Konjugats nach Anspruch 11 als Desinfektionsmittel zu nicht-medizinischen Zwecken.

14. Verwendung des Hydrogels nach einem der Ansprüche 1 bis 10 oder des Konjugats nach Anspruch 11 zur Herstellung eines Desinfektionsreiniger, insbesondere zur Reinigung und Pflege von Horn bei Tieren.

15. Verwendung des Hydrogels nach einem der Ansprüche 1 bis 10 oder des Konjugats nach Anspruch 11 als Desinfektionsreiniger, insbesondere zur Reinigung und Pflege von Horn bei Tieren.

16. Verwendung nach einem der Ansprüche 12 bis 15, wobei das Konjugat in einer Salbe oder einer Creme eingearbeitet vorliegt.

17. Verfahren zur Herstellung des Hydrogels nach einem der Ansprüche 1 bis 10, **dadurch gekennzeichnet, daß** aus Wasser und Verdicker ein Hydrogel hergestellt und diesem bei einer Temperatur von 20 bis 60°C zunächst der Polyalkohol und schließlich das Triiodmethan in Lösevermittler zugesetzt wird.

18. Verfahren nach Anspruch 17, **dadurch gekennzeichnet, daß** das Hydrogel im Anschluß homogenisiert wird, vorzugsweise bei einer Temperatur von 20 bis 40°C.

19. Verfahren nach Anspruch 17 oder 18, **dadurch gekennzeichnet, daß** aus dem Hydrogel, insbesondere durch Fällung mittels hoher Kochsalzkonzentration oder durch hohe Wasserverdünnung, ein Konjugat abgetrennt wird.

## Claims

1. Hydrogel containing a conjugate of triiodomethane and at least one natural organic and/or modified natural organic and/or inorganic thickener as well as water and a solubility promoter wherein the hydrogel has a viscosity between 5,000 and 400,000 mPa*s (20°C), preferably between 10,000 and 30,000 mPa*s (20°C).

2. Hydrogel according to claim 1, **characterised in that** the thickener is a modified natural organic thickener, in particular a cellulose ether or starch ether, preferably methylcellulose, ethylcellulose, methylhydroxyethylcellulose, methylhydroxypropylcellulose, hydroxyethylcellulose, hydroxypropylcellulose, ethylhydroxyethylcellulose, carboxymethylcellulose, hydroxypropyl starch ether, hydroxyethyl starch ether or carboxymethyl starch ether.

3. Hydrogel according to claim 1, **characterised in that** the thickener is a natural organic thickener, in particular agar-agar, guar meal, locust tree meal or starch.

4. Hydrogel according to claim 1, **characterised in that** the thickener is an inorganic thickener, in particular a polysilicic acid or a clay mineral, preferably bentonite.

5. Hydrogel according to one of claims 1 to 4, **characterised in that** the solubility promoter is a a glycol ether, in particular a monomethyl, monoethyl, monobutyl, dimethyl, diethyl or dibutyl ether of ethylene glycol, diethylene glycol, triethylene glycol, propylene glycol, dipropylene glycol or tripropylene glycol, in particular dipropylene glycol-mono-methyl ether or dipropylene glycol-dimethyl ether.

6. Hydrogel according to one of claims 1 to 5, **characterised in that** the hydrogel in case of a natural organic thickener contains 0.3 to 15 wt.%, preferably 0.5 to 10 wt.% of thickener, in case of a modified natural organic thickener 0.5 to 20 wt.%, preferably 1 to 15 wt.% and in case of an inorganic thickener 5 to 25 wt.%, preferably 10 to 20 wt.% of the thickenener, 0.5 to 30 wt.%, preferably 2 to 10 wt.% of solubility promoter and 0.01 to 20 wt.%, preferably 0.1 to 15 wt.% of triiodomethane.

7. Hydrogel according to one of claims 1 to 6, **characterised in that** the hydrogel also contains at least one polyalcohol, in particular ethylene glycol, diethylene glycol, triethylene glycol, 1,2-propylene glycol, dipropylene glycol, tripropylene glycol or glycerol.

8. Hydrogel according to claim 7, **characterised in that** the hydrogel contains 0.01 to 40 wt.%, preferably 5 to 30 wt.%, polyalcohol.

9. Hydrogel according to one of claims 1 to 8, **characterised in that** the hydrogel also contains 1 to 10 wt.%, preferably 3 to 8 wt.%, of a long-chain ester, in particular a triglyceride, preferably laurel oil.

10. Hydrogel according to one of claims 1 to 9, **characterised in that** the hydrogel also contains 0.01 to 0.1 wt.% of perfume oils.

11. Conjugate of triiodomethane and at least one natural organic and/or modified natural organic and/or inorganic thickener, achievable by precipitation of the hydrogel according to one of claims 1 to 10.

12. Use of the hydrogel, according to one of claims 1 to 10 or the conjugate according to claim 11 for preparation of a disinfecting agent.

13. Use of the hydrogel, according to one of claims 1 to 10 or the conjugate according to claim 11 as disinfecting agent for non-medical purposes.

14. Use of the hydrogel, according to one of claims 1 to 10 or the conjugate according to claim 11 for preparation of a disinfectant cleaner, in particular for cleaning and care of animal horn.

15. Use of the hydrogel, according to one of claims 1 to 10 or the conjugate according to claim 11 as disinfectant cleaner, in particular for cleaning and care of animal horn.

16. Use according to claims 12 to 15, wherein the conjugate is present incorporated in an ointment or a cream.

17. Process for the production of the hydrogel according to one of claims 1 to 10, **characterised in that** a hydrogel is produced from water and thickener and to this is added at a temperature of 20 to 60°C, first of all the polyalcohol and then the triiodomethane in solubility promoter.

18. Process according to claim 17, **characterised in that** the hydrogel is then homogenised, preferably at a temperature of 20 to 40°C.

19. Process according to claim 17 or 18, **characterised in that** a conjugate is separated off from the hydrogel, in particular by precipitation by means of high sodium chloride concentration or by high water dilution.

## Revendications

1. Hydrogel, contenant un conjugué de trüodméthane et au moins un épaississant naturel organique et/ou modifié naturel organique et/ou anorganique, ainsi que de l'eau et un émulsifiant, l'hydrogel ayant une viscosité comprise entre 5 000 et 400 000 mPa*s (20°C), de préférence entre 10 000 et 30 000 mPa*s (20°C).

2. Hydrogel selon la revendication 1, **caractérisé en ce que** l'épaississant est un épaississant modifié naturel organique, en particulier une cellulose d'éther ou un éther d'amidon, de préférence le cellulose méthyle, l'éthyle cellulose, le methyl-hydroxyéthyl-cellulose, le methyl-hydroxypropyl cellulose, l'hydroxyethyl cellulose, l'hydroxy-propyl cellulose, l'ethylhydroxyéthyl cellulose, le carboxymethyl cellulose, l'hydroxypropyl éther d'amidon, l'hydroxyéthyl éther d'amidon ou le carboxymethyl éther d'amidon.

3. Hydrogel selon la revendication 1, **caractérisé en ce que** l'épaississant est un épaississant naturel organique, en particulier l'Agar-agar, la farine de graines de guar, la farine de graine de caroubier, ou l'amidon.

4. Hydrogel selon la revendication 1, **caractérisé en ce que** l'épaississant est un épaississant anorganique, en particulier un acide polysilicique ou un minéral d'argile, de préférence la bentonite.

5. Hydrogel selon l'une des revendications 1 à 4, **caractérisé en ce que** l'émulsifiant est un éther de glycol, en particulier un monométhyl, monoéthyl, monobutyl, diméthyl, diéthyl ou dibutyl éther d'éthylène glycol, diethylenglycol, triethylenglycol, propylenglycol ou dipropylenglycol ou tripropylenglycol, en particulier le dipropylenglycol-mono-methylether ou le dipropylenglycol-dimethylether.

6. Hydrogel selon l'une des revendications 1 à 5, **caractérisé en ce que** l'hydrogel, dans le cas d'un épaississant naturel organique, contient de 0,3 à 15 % du poids, de préférence de 0,5 à 10 % du poids, d'épaississant, dans le cas d'un épaississant modifié naturel organique, il contient de 0,5 à 20 % du poids, de préférence de 1 à 15 % du poids d'épaississant, et dans le cas d'un épaississant anorganique, de 5 à 25 % du poids, de préférence de 10 à 20 % du poids d'épaississant, de 0,5 à 30 % du poids, de préférence de 2 à 10 % du poids d'émulsifiant, et de 0,01 à 20 % du poids, de préférence de 0,1 à 15 % du poids de triiodméthane.

7. Hydrogel selon l'une des revendications 1 à 6, **caractérisé en ce que** l'hydrogel contient en outre au moins un poly-alcool, en particulier l'éthylène glycol, le diethylenglycol, le triethylenglycol, le 1,2- propylenglycol, le dipropylenglycol, le tripropylenglycol ou de la glycérine.

8. Hydrogel selon la revendication 7, **caractérisé en ce que** l'hydrogel contient de 0,01 à 40 % du poids, de préférence de 5 à 30 % du poids, de poly-alcool.

9. Hydrogel selon l'une des revendications 1 à 8, **caractérisé en ce que** l'hydrogel contient en outre de 1 à 10 % du poids, de préférence de 3 à 8 % du poids, d'un ester à chaîne longue, en particulier d'un triglycéride, de préférence de l'huile de laurier.

10. Hydrogel selon l'une des revendications 1 à 9, **caractérisé en ce que** l'hydrogel contient en outre de 0,01 à 0,1 % du poids d'huiles de parfum.

11. Conjugué de trüodméthane et au moins un épaississant naturel organique et/ou modifié naturel organique et/ ou anorganique, qu'on obtient par précipitation de l'hydrogel selon l'une des revendications de 1 à 10.

12. Utilisation de l'hydrogel selon l'une des revendications de 1 à 10 ou du conjugué selon la revendication 11 pour la fabrication d'un désinfectant.

13. Utilisation de l'hydrogel selon l'une des revendications de 1 à 10 ou du conjugué selon la revendication 11 en tant que désinfectant à des fins non médicales.

14. Utilisation de l'hydrogel selon l'une des revendications de 1 à 10 ou du conjugué selon la revendication 11 pour la fabrication d'un produit nettoyant désinfectant, en particulier pour le nettoyage et le soin des cornes chez les animaux.

15. Utilisation de l'hydrogel selon l'une des revendications de 1 à 10 ou du conjugué selon la revendication 11 en tant que produit nettoyant désinfectant, en particulier pour le nettoyage et le soin des cornes chez les animaux.

16. Utilisation de l'hydrogel selon l'une des revendications de 12 à 15 où le conjugué est intégré dans une pommade ou une crème.

17. Procédé pour la fabrication de l'hydrogel selon l'une des revendications 1 à 10, **caractérisé en ce qu'**un hydrogel est fabriqué à partir d'eau et d'épaississant, et qu'on y rajoute en premier lieu le poly-alcool et finalement le triiodméthane dans l'émulsifiant, à une température de 20 à 60 °C.

18. Procédé selon la revendication 17, **caractérisé en ce que** l'hydrogel est homogénéisé à la fin, de préférence à une température de 20 à 40°C.

19. Procédé selon la revendication 17 ou 18, **caractérisé en ce qu'**un conjugué est séparé à partir de l'hydrogel, en particulier par précipitation au moyen d'une concentration élevée de chlorure de sodium, ou par une dilution poussée dans l'eau.
